# EUROPEAN PATENT SPECIFICATION

(11) **EP 4 261 206 B1**
(45) Date of publication and mention of the grant of the patent: **27.08.2025**
(21) Application number: 21910729.9
(22) Date of filing: 20.12.2021
(51) Int. Cl.: C07C 405/00, B01D 15/32

(54) **METHOD FOR SEPARATING GEOMETRICAL ISOMER**
VERFAHREN ZUR TRENNUNG GEOMETRISCHER ISOMERE
PROCÉDÉ DE SÉPARATION D'ISOMÈRE GÉOMÉTRIQUE

(30) Priority: 23.12.2020 JP 2020214115
(43) Date of publication of application: 18.10.2023
(73) Proprietor: Kyowa Pharma Chemical Co., Ltd., Takaoka-shi, Toyama 933-8511 (JP)
(72) Inventor: YABUUCHI, Yuto, Kawasaki-shi, Kanagawa 210-0003 (JP); TAKEUCHI, Yuki, Takaoka-shi, Toyama 933-8511 (JP)
(74) Representative: Vossius & Partner Patentanwälte Rechtsanwälte mbB
(86) International application number: PCT/JP2021/047078
(87) International publication number: WO 2022/138586

(56) References cited:
- WO-A1-2012/011128
- WO-A1-2015/136317
- WO-A1-2015/136317
- WO-A2-2011/095990
- JP-A- 2010 053 149
- JP-A- 2015 036 382
- JP-A- 2015 172 025
- JP-A- 2015 506 343
- JP-A- 2017 061 523
- JP-B2- 5 209 542

## Description

### Technical Field

The present invention relates to a method for separating geometrical isomer.

### Background Art

Prostaglandins (PGs) are a general term for a group of endogenous bioactive substances that are synthesized in vivo from arachidonic acid by cyclooxygenase metabolism. There are numerous types of prostaglandins and prostaglandin H₂, prostaglandin D₂, prostaglandin E₁, prostaglandin E₂, prostaglandin F₂α, prostaglandin I₂, and the like are known. Prostaglandins are involved in diverse physiological functions via the respective specific G-protein-coupled receptors thereof.

The chemical structure of prostaglandins is characterized by being provided with a cyclopentane ring with four chiral carbons and two aliphatic side chains. For this reason, prostaglandins have long attracted attention as the target of synthetic research or the seeds of drug discovery and various prostaglandin derivatives have been developed so far.

(3aS,4R,5S,6aR)-(+)-hexahydro-5-hydroxy-4-(hydroxymethyl)-2H-cycl openta[b]furan-2-one, used as a common intermediate for the above, is also called "Corey lactone". The chemical structures of Corey lactone and typical commercially available prostaglandin derivatives are shown below.

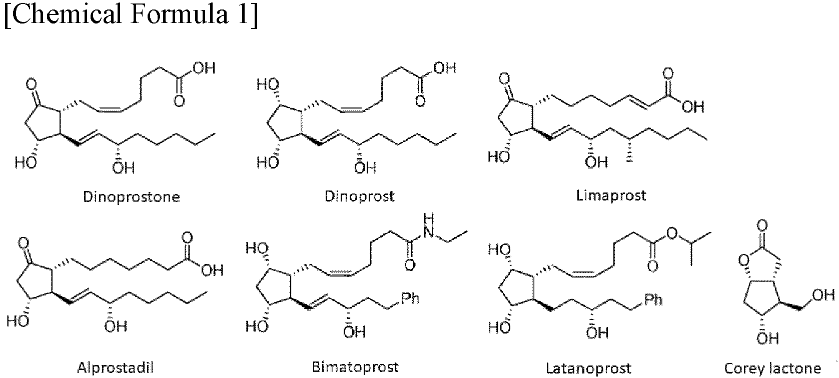

### Citation List

### Patent Literature

[Patent Literature 1] JP 5209542 B2
[Patent Literature 2] WO 2011/095990 A1
[Patent Literature 3] WO 2012/011128 A1
[Patent Literature 4] WO 2015/136317 A1

### Summary of Invention

### Technical Problem

Prostaglandins have a functionalized cyclopentane ring in the center of the chemical structure thereof and long aliphatic side chains on two adjacent carbon atoms, one of which has a carboxy group or carboxylic acid ester. Prostaglandins are generally produced by the following methods, via a common synthetic intermediate, with the oxidation stage of the substituent adjusted in subsequent steps. Many prostaglandins have a cis-type double bond in the aliphatic side chains and the problem is how to remove the geometrical isomers thereof when carrying out the chemical synthesis. Compounds with a cis-type double bond are also called Z-isomers and compounds with a trans-type double bond are called E-isomers.

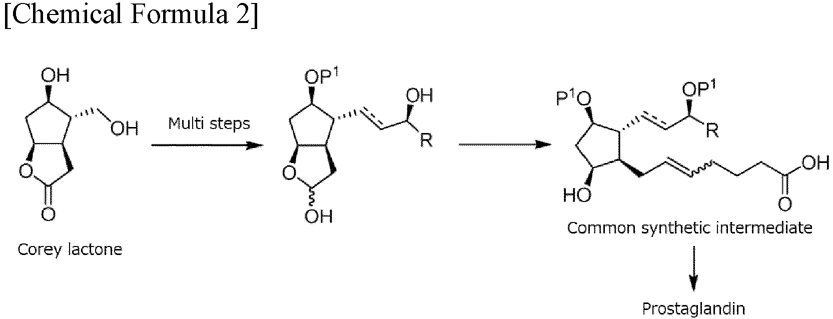

The present invention has an object of providing a method for separating compounds having a structure similar to prostaglandins from the geometrical isomers thereof.

### Solution to Problem

The present invention provides the following [1] to [5].
[1] A method for separating a compound represented by Formula (1) or (2) from a geometrical isomer thereof, in which the geometrical isomer is a geometrical isomer in a double bond included in A, the method including processing a mixture containing the compound and the geometrical isomer thereof by a chromatographic method using an acidic functional group-modified silica gel as a stationary phase. 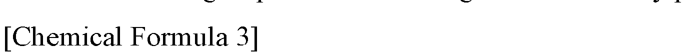 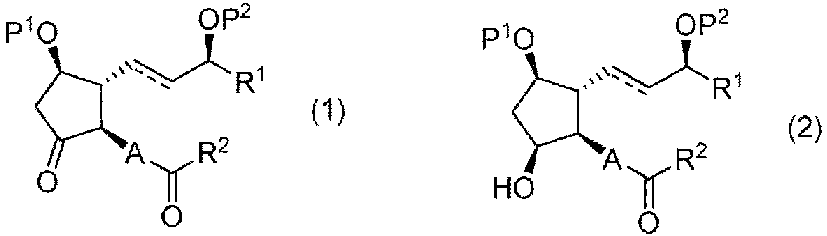 [in the formula, P¹ and P² are each independently a hydrogen atom or a protective group of a hydroxyl group, R¹ is a linear or branched C₁₋₆ alkyl group that may be substituted with a phenyl group, A is a C₃₋₁₀ alkenylene group, R² is a hydroxyl group, a C₁₋₃ alkoxy group, a mono(C₁₋₃ alkyl)amino group, or a di(C₁₋₃ alkyl)amino group, and 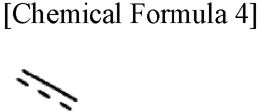 is a single bond or double bond.]
[2] The method according to [1], wherein A is 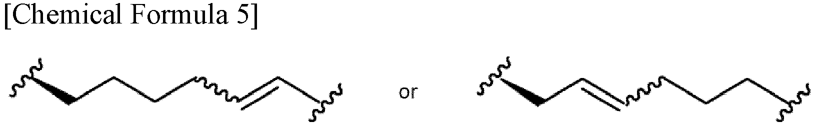
[3] The method according to [1] or [2], wherein R¹ is 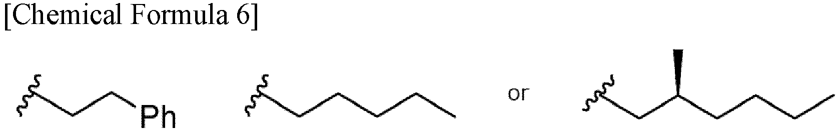
[4] The method according to any one of [1] to [3], wherein P¹ is a hydrogen atom or a 2-tetrahydropyranyl group.
[5] The method according to any one of [1] to [4], wherein the acidic functional group-modified silica gel is a silica gel modified with a carboxy group or a sulfo group.

### Advantageous Effects of Invention

According to the present invention, it is possible to provide a method for separating compounds having a structure similar to prostaglandins from geometrical isomers thereof.

### Description of Embodiments

A detailed description will be given below of the present invention.

One embodiment of the present invention is method for separating a compound represented by Formula (1) or (2) from a geometrical isomer thereof, in which the geometrical isomer is a geometrical isomer in a double bond included in A, the method including processing a mixture containing the compound and the geometrical isomer thereof by a chromatographic method using an acidic functional group-modified silica gel as a stationary phase. 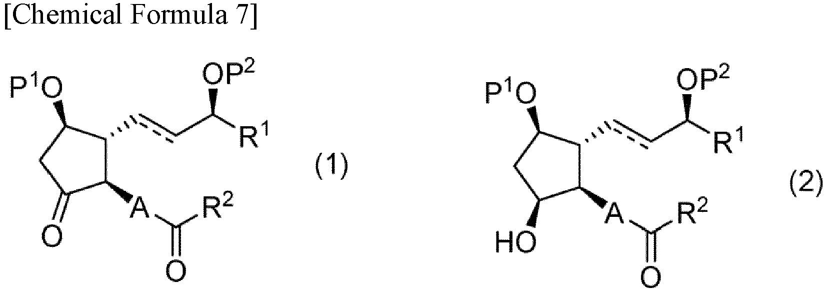 [in the formula, P¹ and P² are each independently a hydrogen atom or a protective group of a hydroxyl group, R¹ is a linear or branched C₁₋₆ alkyl group that may be substituted with a phenyl group, A is a C₃₋₁₀ alkenylene group, R² is a hydroxyl group, a C₁₋₃ alkoxy group, a mono(C₁₋₃ alkyl)amino group, or a di(C₁₋₃ alkyl)amino group, and 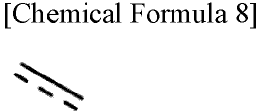 is a single bond or double bond.]

The combination of two or more geometrical isomers as separation targets in the method according to the present embodiment is a compound represented by Formula (1) or (2), which is in a cis-isomer and trans-isomer relationship in the double bond included in A. In a case where the double bond is also present in the other side chain (the side chain having R¹), it is possible for a total of four types of geometrical isomers to be present.

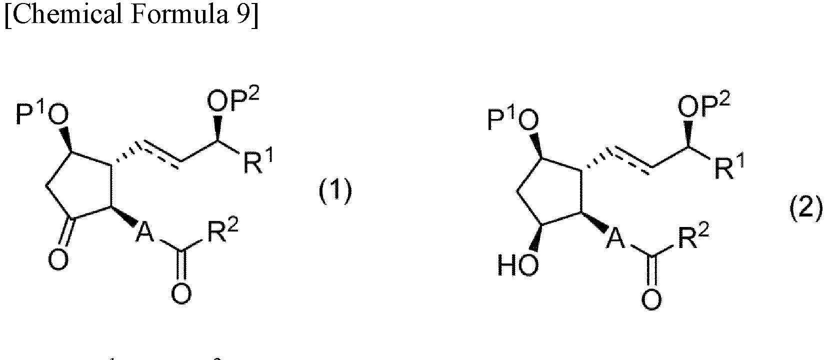

P¹ and P² are each independently a hydrogen atom or a protective group of a hydroxyl group. The protective group of the hydroxyl group is a substituent used for the purpose of protecting the hydroxyl group to prevent reaction with a reactant in an organic synthetic reaction. The protective group of the hydroxyl group is not particularly limited and examples thereof include acetal-based protective groups such as a methoxymethyl group, an ethoxyethyl group, a benzyloxymethyl group, or a tetrahydropyranyl group, ether-based protective groups such as a benzyl group, a p-methoxybenzyl group, or a p-nitrobenzyl group, acyl-based protective groups such as an acetyl group, a pivaloyl group, a benzoyl group, and a p-methoxybenzoyl group, and silyl-based protective groups such as a trimethylsilyl group, a triethylsilyl group, a tert-butyldimethyl group, a triphenylsilyl group, and a phenyldimethylsilyl group.

R¹ is a linear or branched C₁₋₆ alkyl group that may be substituted with a phenyl group. A linear or branched C₁₋₆ alkyl group is an alkyl group having 1 to 6 carbon atoms and specific examples thereof include a methyl group, an ethyl group, a 1-propyl group, a 2-propyl group, a 1-butyl group, a 2-butyl group, a tert-butyl group, a 1-pentyl group, a 2-pentyl group, a 3-pentyl group, a 1,1-dimethyl propyl group, 1-hexyl group, 2-hexyl group, a 3-hexyl group, and the like. R¹ may be the linear or branched C₁₋₆ alkyl group described above substituted with a phenyl group.

A is a C_{3-1O} alkenylene group and specific examples thereof include a propenylene group, a butenylene group, a pentenylene group, a hexenylene group, a heptenylene group, an octenylene group, a nonylene group, or a decenylene group. The position of the double bond in A is not particularly limited. According to the separation method according to the present embodiment, it is possible to separate the geometrical isomers (cis-isomer and trans-isomer) in the double bond.

R² is a hydroxyl group, a C₁₋₃ alkoxy group, a mono(C₁₋₃ alkyl)amino group or a di(C₁₋₃ alkyl)amino group. A C₁₋₃ alkoxy group is a group in which an alkyl group having 1 to 3 carbon atoms is substituted for an oxygen atom and specific examples thereof include a methoxy group, an ethoxy group, a 1-propoxy group, or a 2-propoxy group. A mono(C₁₋₃ alkyl)amino group is a group in which one alkyl group having 1 to 3 carbon atoms is substituted for a nitrogen atom and specific examples thereof include a monomethylamino group, a monoethylamino group, a mono(1-propyl)amino group, a mono(2-propyl)amino group, and the like. A di(C₁₋₃ alkyl)amino group is a group in which two alkyl groups having 1 to 3 carbon atoms are substituted for a nitrogen atom and specific examples thereof include a dimethyl amino group, a diethyl amino group, a di(1-propyl)amino group, a di(2-propyl)amino group, an ethyl(methyl)amino group, and the like.

The separation method according to the present embodiment includes treating the mixture of geometrical isomers by a chromatographic method using an acidic functional group-modified silica gel as a filling material (stationary phase).

The acidic functional group-modified silica gel used as the stationary phase in the chromatographic method may be any silica gel modified with acidic functional groups. Examples of acidic functional group-modified silica gels include carboxy group-modified silica gels, sulfo group-modified silica gels, and the like. The chromatographic method described above is preferably normal-phase chromatography.

The shape of the acidic functional group-modified silica gel may be spherical or crushed, preferably spherical. Spherical silica gel has a constant surface area and is able to be packed uniformly in a column, thus, the degree of separation is further improved when carrying out separation by the chromatographic method.

The average particle size of the acidic functional group-modified silica gel may be 3 µm to 500 µm, preferably 5 µm to 300 µm, and more preferably 30 µm to 200 µm.

The length of the column may be 5 cm to 200 cm, preferably 10 cm to 150 cm, and more preferably 15 cm to 100 cm. A column length of 15 cm or longer improves the degree of separation and widens the eluent selection range.

For the eluent (mobile phase), it is possible to use organic solvents well known to a person skilled in the art. Examples of organic solvents include aliphatic hydrocarbon-based solvents such as pentane, hexane, and heptane; aromatic hydrocarbon-based solvents such as toluene; halogenated hydrocarbon-based solvents such as dichloromethane and chloroform; ester-based solvents such as ethyl acetate and propyl acetate; alcohol-based solvents such as methanol, ethanol, and 2-propanol, and the like. It is possible to select these solvents as appropriate in consideration of the solubility of the crude product for purification which is the separation target and also to mix and use these solvents in any ratio in consideration of mutual compatibility. Examples of mixed solvents include binary mixed solvents such as a combination of hexane and ethanol, hexane and isopropanol, or hexane and ethyl acetate, and ternary mixed solvents such as a combination of hexane, methanol, and isopropanol.

In the present specification, "separating a compound represented by Formula (1) or (2) from the geometrical isomer thereof" means that, in a case where the content of the desired compound is 100, the content of the corresponding geometrical isomer is 2 or less, preferably 1 or less, and more preferably 0.5 or less. In addition, the purity of the compound represented by Formula (1) or (2) after separation may be 90% or more, 95% or more is preferable, and 97% or more, 98% or more, or 99% or more is more preferable.

### [Examples]

A more detailed description will be given below of the present invention using Examples and Comparative Examples.

Abbreviations used in the Examples and the like are to be understood with the meanings well-known to a person skilled in the art, unless otherwise noted. For example, the meanings of some abbreviations are given below.
THP: 2-tetrahydropyranyl
Ph: phenyl

### Example 1

### Purification of (Z)-7-[(1R,2R,3R,5 S)-3,5-dihydroxy-2-[(3R)-3-hydroxy-5-phenylpentyl ]cyclopentyl]hepto-5-enoic acid ((Z)-IFL-FA)

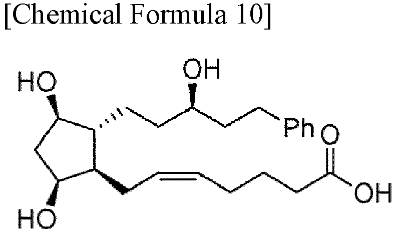

(Z)-IFL-FA

### Example 1a

A crude product for purification (EZ mixture of IFL-FA, 238 mg) was dissolved in dichloromethane and purified by a chromatographic method according to the following separation conditions and the fractions where the E-isomer was not detected under the following analysis conditions were collected to obtain the Z-isomer (yield amount: 206 mg, yield rate: 90%).

### <Separation Conditions>

Eluent: Hexane:Isopropanol = 10:1
Filling material: CROMATOREX COOH MB100-40/75 (trade name, manufactured by Fuji Silysia Chemical Ltd., spherical silica gel (average particle size 40 µm to 75 µm, pore diameter 10 nm)) Preparation volume: 1 to 2 mL/fraction
<Analysis Conditions>
Column: YMC-Pack SIL (trade name, manufactured by YMC Co., Ltd., inner diameter: 4.6 mm, length: 25 cm)
Mobile phase: Hexane:Ethanol:Acetic acid = 91:9:0.05
Flow rate: 1 mL per minute
Detection wavelength: 215 nm
Injection volume: 20 µL

The content ratio of each isomer before and after separation is shown in Table 1. The content ratio of each isomer was calculated based on the area under the curve of the chromatogram obtained under the analysis conditions described above.
(E)-IFL-FA is (E)-7-[(1R,2R,3R,5S)-3,5-dihydroxy-2-[(3R)-3-hydroxy-5-phenylpentyl ]cyclopentyl]hepto-5-enoic acid.

**[Table 1]**

| | Before separation | After separation |
|---|---|---|
| (Z)-IFL-FA | 74.0% | 82.0% |
| (E)-IFL-FA | 1.1% | 0.0% |

### Example 1b

A crude product for purification (EZ mixture of IFL-FA, 224 mg) was dissolved in dichloromethane and purified by a chromatographic method according to the following separation conditions and the fractions where the E-isomer was not detected under the following analysis conditions were collected to obtain the Z-isomer (yield amount: 142 mg, yield rate: 68%).

### <Separation Conditions>

Eluent: Hexane:Isopropanol = 10:1
Filling material: CROMATOREX SO3H MB100-40/75 (trade name, manufactured by Fuji Silysia Chemical Ltd., spherical silica gel (average particle size 40 µm to 75 µm, pore diameter 10 nm))
Preparation volume: 1 to 2 mL/fraction
<Analysis Conditions>
Column: YMC-Pack SIL (trade name, manufactured by YMC Co., Ltd., inner diameter: 4.6 mm, length: 25 cm)
Mobile phase: Hexane: Ethanol: Acetic acid = 91:9:0.05
Flow rate: 1 mL per minute
Detection wavelength: 215 nm
Injection volume: 20 µL

The content ratio of each isomer before and after separation is shown in Table 2. The content ratio of each isomer was calculated based on the area under the curve of the chromatogram obtained under the analysis conditions described above. The content ratio of the E-isomer was 0.0% and the Z-isomer was obtained with high purity.

**[Table 2]**

| | Before separation | After separation |
|---|---|---|
| (Z)-IFL-FA | 74.0% | 99.2% |
| (E)-IFL-FA | 1.1% | 0.0% |

### Example 2

### Purification of (Z)-7-[(1R,2R,3R,5S)-5-hydroxy-2-[(3R)-5-phenyl-3-[(tetrahydro-2H-p yran-2-yl)oxy]pentyl]-3-[(tetrahydro-2H-pyran-2-yl)oxy]cyclopentyl]he pto-5-enoic acid ((Z)-IFL-PPF)

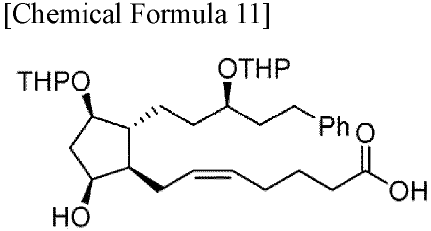

### (Z)-IFL-PPF

A crude product for purification (EZ mixture of IFL-PPF, 392 mg) was dissolved in a mixture of hexane and ethyl acetate and purified by a chromatographic method according to the following separation conditions and the fractions having 0.5% or less of the E-isomer were collected under the following analysis conditions to obtain the Z-isomer (yield amount: 99 mg, yield rate: 68%). Analysis was performed after concentrating approximately 0.1 mL of the fraction, dissolving the residue in 1 mL of 2-propanol, adding a catalytic amount of paratoluenesulfonic acid monohydrate and carrying out a reaction at room temperature for approximately 2 hours to deprotect and derivatize to IFL-FA (refer to Example 1).

### <Separation Conditions>

Eluent: Hexane:ethyl acetate = 3:1-3:7
Filling material: CROMATOREX SO3H MB100-40/75 (trade name, manufactured by Fuji Silysia Chemical Ltd., spherical silica gel (average particle size: 40 µm to 75 µm, pore diameter 10 nm))
Preparation volume: 1 to 2 mL/fraction
<Analysis Conditions>
Column: YMC-Pack SIL (trade name, manufactured by YMC Co., Ltd., inner diameter: 4.6 mm, length: 25 cm)
Mobile phase: Hexane:Ethanol:Acetic acid = 91:9:0.05
Flow rate: 1 mL per minute
Detection wavelength: 215 nm
Injection volume: 20 µL

The content ratio of each isomer before and after separation is shown in Table 3. The content ratio of each isomer was calculated based on the area under the curve of the chromatogram. (E)-IFL-PPF is (E)-7-[(1R,2R,3R,5S)-5-hydroxy-2-[(3R)-5-phenyl-3-[(tetrahydro-2H-p yran-2-yl)oxy]pentyl]-3-[(tetrahydro-2H-pyran-2-yl)oxy]cyclopentyl]he pto-5-enoic acid. The E-isomer included in the Z-isomer after separation was 0.2% when measured under the above analysis conditions. In addition, it was also possible to separate triphenylphosphine, which was included to a large extent in the crude product for purification.

**[Table 3]**

| | Before separation | After separation |
|---|---|---|
| (Z)-IFL-PPF | 17.3% | 99.2% |
| (E)-IFL-PPF | 2.0% | 0.2% |
| Triphenylphosphine oxide | 78.0% | 0.5% |

### Example 3

### Purification of propan-2-yl (Z)-7-[(1R,2R,3R,5 S)-3,5-dihydroxy-2-[(3R)-3-hydroxy-5-phenylpentyl ]cyclopentyl]hepto-5-enoate ((Z)-latanoprost)

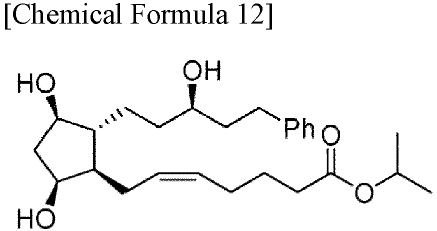

### (Z)-IFL

### Example 3a

The crude product for purification (EZ mixture of latanoprost, 200 mg) was dissolved in a mixture of hexane and ethyl acetate and purified by the chromatographic method according to the following separation conditions and the fractions having 0.5% or less of the E-isomer were collected under the following analysis conditions to obtain the Z-isomer (yield amount: 94 mg, yield rate: 47%).

### <Separation Conditions>

Eluent: Hexane:Ethyl acetate = 3:1-3:7
Filling material: CROMATOREX COOH MB100-40/75 (trade name, manufactured by Fuji Silysia Chemical Ltd., spherical silica gel (average particle size: 40 µm to 75 µm, pore diameter: 10 nm))
Preparation volume: 1 to 2 mL/fraction
<Analysis conditions>
Column: Spherisorb Silica (trade name, manufactured by Waters Corporation, inner diameter: 4.6 mm, length: 25 cm)
Mobile phase: Hexane:Ethanol:Acetic acid = 91:9:0.05
Flow rate: 1 mL per minute
Detection wavelength: 215 nm
Injection volume: 20 µL

The content ratio of each isomer before and after separation is shown in Table 4. The content ratio of each isomer was calculated based on the area under the curve of the chromatogram. (E)-Latanoprost is a propan-2-yl (E)-7-[(1R,2R,3R,5S)-3,5-dihydroxy-2-[(3R)-3-hydroxy-5-phenylpentyl ]cyclopentyl]hepto-5-enoate. The content ratio of the E-isomer was 0.4% under the above analysis conditions and it was possible to obtain the Z-isomer with high purity.

**[Table 4]**

| | Before separation | After separation |
|---|---|---|
| (Z)-Latanoprost | 96.3% | 98.7% |
| (E)-Latanoprost | 1.5% | 0.4% |

### Example 3b

The crude product for purification (EZ mixture of IFL, 200 mg) was dissolved in a mixture of hexane and ethyl acetate and purified by the chromatographic method according to the following separation conditions and the fractions having 0.5% or less of the E-isomer were collected under the following analysis conditions to obtain the Z-isomer (yield amount: 64 mg, yield rate: 32%).

### <Separation Conditions>

Eluent: Hexane:Ethyl acetate = 3:1 to 2:3
Filling material: CROMATOREX SO3H MB100-40/75 (trade name, manufactured by Fuji Silysia Chemical Ltd., spherical silica gel (average particle size: 40 µm to 75 µm, pore diameter: 10 nm))
Preparation volume: 1 to 2 mL/fraction
<Analysis conditions>
Column: Spherisorb Silica (trade name, manufactured by Waters Corporation, inner diameter: 4.6 mm, length: 25 cm)
Mobile phase: Hexane:Ethanol:Acetic acid = 91:9:0.05
Flow rate: 1 mL per minute
Detection wavelength: 215 nm
Injection volume: 20 µL

The content ratio of each isomer before and after separation is shown in Table 5. The content ratio of each isomer was calculated based on the area under the curve of the chromatogram. The content ratio of the E-isomer was 0.5% and it was possible to obtain the Z-isomer with high purity.

**[Table 5]**

| | Before separation | After separation |
|---|---|---|
| (Z)-Latanoprost | 98.2% | 99.5% |
| (E)-Latanoprost | 1.6% | 0.5% |

### Example 4

### Purification of (E)-7-((1R,2R,3R)-3-hydroxy-2-((3 S,5 S,E)-3-hydroxy-5-methylnon-1-e n-1-yl)-5-oxocyclopentyl)hepto-2-enoic acid ((E)-IEL)

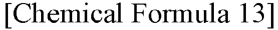

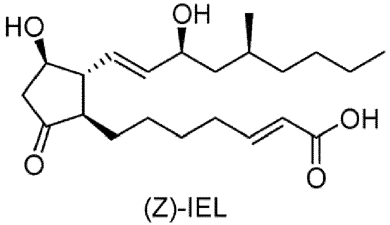

The crude product for purification (EZ mixture of IEL, 33 mg) was dissolved in dichloromethane and purified by the chromatographic method according to the following separation conditions and the fractions where the E-isomer was not detected under the following analysis conditions were collected to obtain the E-isomer (yield amount: 13 mg, yield rate: 56%).

### <Separation Conditions>

Eluent: Hexane:Ethanol = 10:1
Filling material: CROMATOREX COOH SMB100-10 (trade name, manufactured by Fuji Silysia Chemical Ltd., Spherical silica gel (average particle size: 10 µm, pore diameter: 10 nm))
Preparation volume: 1 to 2 mL/fraction
<Analysis conditions>
Column: Develosil ODS-5 (trade name, manufactured by Nomura Chemical Co., Ltd., inner diameter: 4.6 mm, length: 15 cm)
Mobile phase: 0.02 M potassium dihydrogen phosphate buffer: acetonitrile: 2-propanol = 9:5:2
Flow rate: 1 mL per minute
Detection wavelength: 215 nm
Injection volume: 20 µL

The content ratios of each isomer before and after separation are shown in Table 6. The content ratio of each compound was calculated based on the area under the curve of the chromatogram. (Z)-IEL is (Z)-7-((1R,2R,3R)-3-hydroxy-2-((3S,5S,E)-3-hydroxy-5-methylnon-1-e n-1-yl)-5-oxocyclopentyl)hepto-2-enoic acid, and AT-IEL is (E)-7-((1R,2S)-2-((3S,5S,E)-3-hydroxy-5-methylnon-1-en-1-yl)-5-oxoc yclopent-3-en-1-yl)hepto-2-enoic acid. The content ratio of the E-isomer included in the Z-isomer after separation was 0.0% under the above analysis conditions. In addition, it was also possible to separate AT-IEL, which was included to a large extent in the crude product for purification.

**[Table 6]**

| | Before separation | After separation |
|---|---|---|
| (E)-IEL | 74.8% | 93.6% |
| (Z)-IEL | 1.6% | 0.0% |
| AT-IEL | 10.0% | 0.4% |

### Comparative Example 1

### Purification of (Z)-7-[(1R,2R,3R,5S)-5-hydroxy-2-[(3R)-5-phenyl-3-[(tetrahydro-2H-p yran-2-yl)oxy]pentyl]-3-[(tetrahydro-2H-pyran-2-yl)oxy]cyclopentyl]he pto-5-enoic acid ((Z)-IFL-PPF)

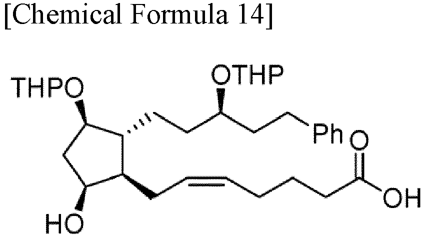

### (Z)-IFL-PPF

The crude product for purification (EZ mixture, 75.8 g) was dissolved in a mixed solvent of hexane and ethyl acetate and purified by the chromatographic method according to the following separation conditions to obtain the (Z) isomer (yield amount: 45.3 g, yield rate: 96%). In all recovered fractions, (E)-latanoprost was detected and was not able to be separated. Analysis was performed by concentrating approximately 0.1 mL of the fractions, dissolving the residue in 1 mL of 2-propanol, adding a catalytic amount of paratoluenesulfonic acid monohydrate, and carrying out a reaction at room temperature for approximately 2 hours to deprotect and derivatize to IFL-FA (refer to Example 1).

### <Separation Conditions>

Eluent: Hexane:ethyl acetate = 3:2
Filling material: BW-300S (trade name, manufactured by Fuji Silysia Chemical Ltd., crushed silica gel (average particle size: 38 µm to 75 µm, pore diameter: 6 nm))
Preparation volume: 20-50 mL/fraction
<Analysis Conditions>
Column: YMC-Pack SIL (trade name, manufactured by YMC Co., Ltd., inner diameter: 4.6 mm, length: 25 cm)
Mobile phase: Hexane:Ethanol:Acetic acid = 91:9:0.05
Flow rate: 1 mL per minute
Detection wavelength: 215 nm
Injection volume: 20 µL

The content ratio of each isomer before and after separation is shown in Table 7. The content ratio of each isomer was calculated based on the area under the curve of the chromatogram.

**[Table 7]**

| | Before separation | After separation |
|---|---|---|
| (Z)-IFL-PPF | 63% | 97.2% |
| (E)-IFL-PPF | 1.9% | 1.9% |

### Comparative Example 2

### Purification of propan-2-yl (Z)-7-[(1R,2R,3R,5 S)-3,5-dihydroxy-2-[(3R)-3-hydroxy-5-phenylpentyl ]cyclopentyl]hepto-5-enoate ((Z)-latanoprost)

The crude product for purification (EZ mixture of latanoprost, 17.3 g) was dissolved in a mixture of hexane and ethyl acetate and purified by the chromatographic method according to the following separation conditions to obtain (Z)-latanoprost (yield amount: 14.6 g, yield rate: 88%). In all fractions including (Z)-latanoprost, (E)-latanoprost was detected under the following analysis conditions and was not able to be separated.

### <Separation Conditions>

Eluent: Hexane:Ethyl acetate = 3:2
Filling material: Silica gel 60 high-purity product (trade name, manufactured by Kanto Chemical Co., Inc.)
Preparation volume: 20-50 ml/fraction
<Analysis Conditions>
Column: Spherisorb Silica (trade name, manufactured by Waters Corporation, inner diameter: 4.6 mm, length: 25 cm)
Mobile phase: Hexane:Ethanol:Acetic acid = 91:9:0.05
Flow rate: 1 mL per minute
Detection wavelength: 215 nm
Injection volume: 20 µL

The content ratio of each isomer before and after separation is shown in Table 8. The content ratio of each isomer content was calculated based on the area under the curve of the chromatogram.

**[Table 8]**

| | Before separation | After separation |
|---|---|---|
| (Z)-Latanoprost | 95.5% | 98.1% |
| (E)-Latanoprost | 1.9% | 1.9% |

### Reference Example 1

### Purification of (Z)-7-((1R,2R,3R)-3-hydroxy-2-((S,E)-3-hydroxyocto-1-en-1-yl)-5-oxo cyclopentyl)hepto-5-enoic acid (PGE₂, dinoprostone).

The crude product for purification (mixture of PGE₂ and PGA₂, 35 mg) was dissolved in dichloromethane and purified by the chromatographic method according to the following separation conditions and the fractions not including PGA2 were collected under the following analysis conditions to obtain PGE2 (yield amount: 20 mg, yield rate: 100%).

### <Separation Conditions>

Eluent: Hexane:Ethanol = 10:1
Filling material: CROMATOREX COOH SMB100-10 (trade name, manufactured by Fuji Silysia Chemical Ltd., Spherical silica gel (average particle size: 10 µm, pore diameter: 10 nm))
Preparation volume: 1 to 2 mL/fraction.

The content ratios of each isomer before separation and after separation are shown in Table 9. The content ratio of each isomer was calculated based on the area under the curve of the chromatogram obtained under the following analysis conditions.

### <Analysis Conditions>

Column: L-Column 2 ODS (trade name, manufactured by Chemicals Evaluation and Research Institute, Japan, inner diameter: 4.6 mm, length: 25 cm)
Mobile phase: Methanol:0.2 volume% acetic acid solution = 58:42
Flow rate: 1 mL per minute
Detection wavelength: 210 nm
Injection volume: 20 µL

**[Table 9]**

| | Before separation | After separation |
|---|---|---|
| PGE₂ | 71.2% | 93.6% |
| PGA₂ | 13.5% | 1.1% |

## Claims

1. A method for separating a compound represented by Formula (1) or (2) from a geometrical isomer thereof, wherein the geometrical isomer is a geometrical isomer in a double bond included in A, the method comprising:
processing a mixture containing the compound and the geometrical isomer thereof by a chromatographic method using an acidic functional group-modified silica gel as a stationary phase, 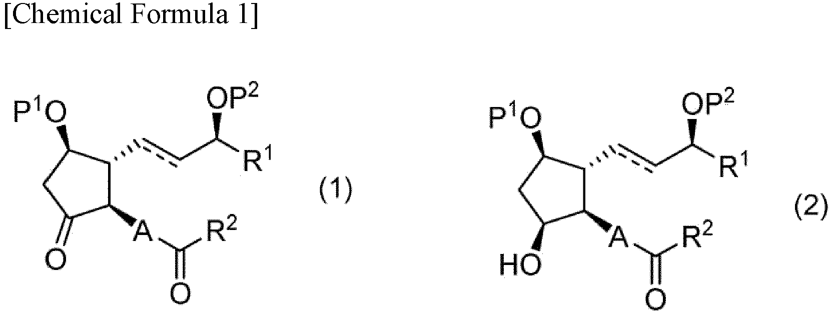 in the formulae, P¹ and P² are each independently a hydrogen atom or a protective group of a hydroxyl group, R¹ is a linear or branched C₁₋₆ alkyl group that may be substituted with a phenyl group, A is a C₃₋₁₀ alkenylene group, R² is a hydroxyl group, a C₁₋₃ alkoxy group, a mono(C₁₋₃ alkyl)amino group, or a di(C₁₋₃ alkyl)amino group, and 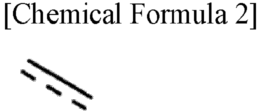 is a single bond or a double bond.

2. The method according to claim 1, wherein A is

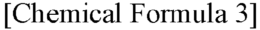

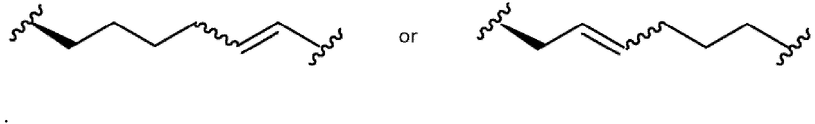

3. The method according to claim 1 or 2, wherein R¹ is

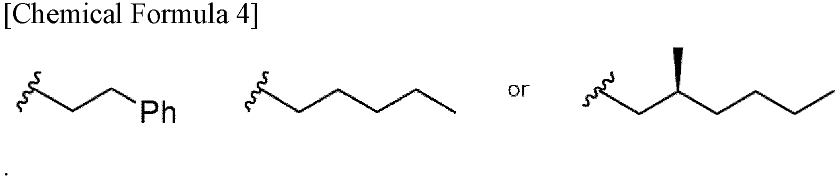

4. The method according to any one of claims 1 to 3, wherein P¹ is a hydrogen atom or a 2-tetrahydropyranyl group.

5. The method according to any one of claims 1 to 4, wherein the acidic functional group-modified silica gel is a silica gel modified with a carboxy group or a sulfo group.

## Patentansprüche

1. Ein Verfahren zum Trennen einer Verbindung, dargestellt durch Formel (1) oder (2), von einem geometrischen Isomer davon, wobei das geometrische Isomer ein geometrisches Isomer in einer Doppelbindung ist, die in A enthalten ist, wobei das Verfahren umfasst:
Verarbeiten eines Gemisches, das die Verbindung und das geometrische Isomer davon enthält, durch ein chromatographisches Verfahren unter Verwendung eines mit einer sauren funktionellen Gruppe modifizierten Kieselgels als stationäre Phase, 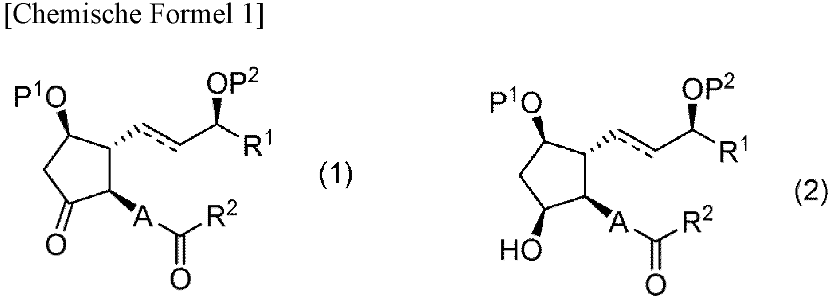 wobei in den Formeln P¹ und P² jeweils unabhängig ein Wasserstoffatom oder eine Schutzgruppe einer Hydroxylgruppe sind, R¹ eine lineare oder verzweigte C₁₋₆-Alkylgruppe ist, die mit einer Phenylgruppe substituiert sein kann, A eine C₃₋₁₀-Alkenylengruppe ist, R² eine Hydroxylgruppe, eine C₁₋₃-Alkoxygruppe, eine Mono(C₁₋₃-alkyl)-aminogruppe oder eine Di(C₁₋₃-alkyl)-aminogruppe ist , und 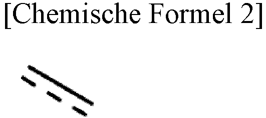 eine Einfachbindung oder eine Doppelbindung ist.

2. Das Verfahren nach Anspruch 1, wobei A 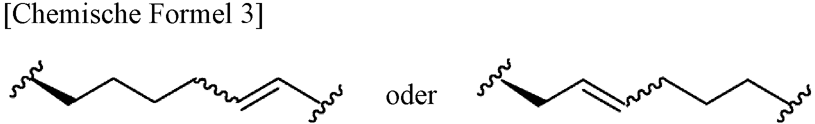 ist.

3. Das Verfahren nach Anspruch 1 oder 2, wobei R¹ 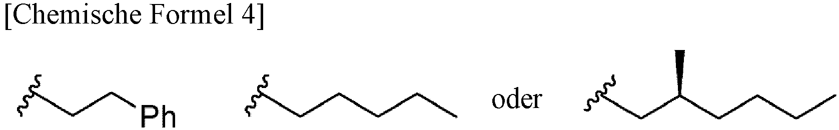 ist.

4. Das Verfahren nach einem der Ansprüche 1 bis 3, wobei P¹ ein Wasserstoffatom oder eine 2-Tetrahydropyranylgruppe ist.

5. Das Verfahren nach einem der Ansprüche 1 bis 4, wobei das mit einer sauren funktionellen Gruppe modifizierte Kieselgel ein mit einer Carboxygruppe oder einer Sulfogruppe modifiziertes Kieselgel ist.

## Revendications

1. Méthode de séparation d'un composé représenté par la formule (1) ou (2) d'un isomère géométrique de celui-ci, dans laquelle l'isomère géométrique est un isomère géométrique au niveau d'une double liaison incluse dans A, la méthode comprenant :
le traitement d'un mélange contenant le composé et l'isomère géométrique de celui-ci par une méthode chromatographique utilisant un gel de silice modifiée par un groupe fonctionnel acide en tant que phase stationnaire, 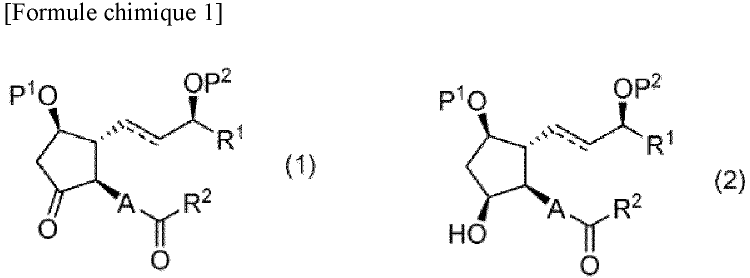
dans les formules, P¹ et P² sont chacun indépendamment un atome d'hydrogène ou un groupe protecteur d'un groupe hydroxyle, R¹ est un groupe alkyle en C₁₋₆ linéaire ou ramifié qui peut être substitué par un groupe phényle, A est un groupe alcénylène en C₃₋₁₀, R² est un groupe hydroxyle, un groupe alcoxy en C₁₋₃, un groupe mono(alkyle en C₁₋₃)amino, ou un groupe di(alkyle en C₁₋₃)amino, et 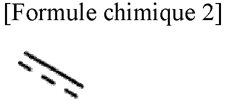
est une liaison simple ou une double liaison.

2. Méthode selon la revendication 1, dans laquelle A est

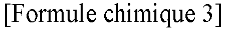

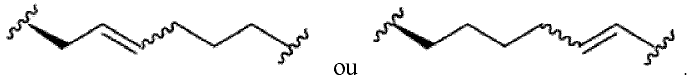

3. Méthode selon la revendication 1 ou 2, dans laquelle R¹ est

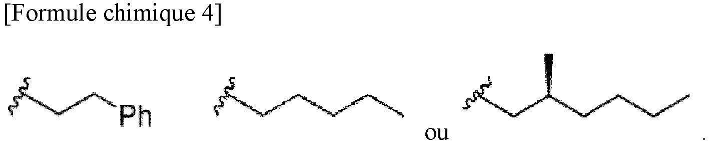

4. Méthode selon l'une quelconque des revendications 1 à 3, dans laquelle P¹ est un atome d'hydrogène ou un groupe 2-tétrahydropyranyle.

5. Méthode selon l'une quelconque des revendications 1 à 4, dans laquelle le gel de silice modifiée par un groupe fonctionnel acide est un gel de silice modifiée par un groupe carboxy ou un groupe sulfo.
